# EUROPÄISCHE PATENTSCHRIFT

(11) **EP 2 025 355 B1**
(45) Veröffentlichungstag und Bekanntmachung des Hinweises auf die Patenterteilung: **30.05.2012**
(21) Anmeldenummer: 08020073.6
(22) Anmeldetag: 16.07.2002
(51) Int. Cl.: A61M 1/16

(54) **Behältnis zur Verwendung in der Dialyse**
Container for use during dialysis
Récipient destiné à l'utilisation en dialyse

(30) Priorität: 23.10.2001 DE 10152105
(43) Veröffentlichungstag der Anmeldung: 18.02.2009
(62) Teilanmeldung aus: 02762365.1
(73) Patentinhaber: Fresenius Medical Care Deutschland GmbH, 61352 Bad Homburg (DE)
(72) Erfinder: Dumon d'Ayot, François, 69005 Lyon (FR); Dupin, Thierry, 69690 Besseway (FR); Laffay, Philippe, 69110 Sainte Fou Les Lyon (FR); Graf, Thomas, Dr., 61352 Bad Homburg (DE)
(74) Vertreter: Laufhütte, Dieter

(56) Entgegenhaltungen:
- EP-A- 0 475 825
- EP-A- 0 484 751
- EP-A- 0 665 026
- FR-A- 2 766 797
- US-A- 5 385 564
- US-A- 5 387 237

## Beschreibung

Die Erfindung betrifft ein Behältnis zur Verwendung in der Dialyse nach dem Oberbegriff des Anspruchs 1.

Bei Dialysesystemen ist es bisher verbreitet, das für den Dialysevorgang notwendige Dialysat erst unmittelbar vor der Dialysesitzung herzustellen. Hierzu wird in einem Behältnis das Dialysatkonzentrat in fester Form vorgelegt, wobei dieses üblicherweise in Form von Pulvern, Granulaten oder Pastillen, d.h. Presslingen aus Pulver, vorgelegt wird. Zur Bildung des Dialysats muß das feste Dialysatkonzentrat in Wasser gelöst werden. Hierzu wird das Behältnis mit dem festen Dialysatkonzentrat in die Dialysiervorrichtung eingespannt und üblicherweise wird das Wasser, in welchem das Dialysatkonzentrat aufgelöst werden soll, auf einer Seite in das Behältnis eingeleitet und auf der gegenüberliegenden Seite wieder abgezogen. Hierbei ergibt sich die Problematik, daß die abgezogene Lösung nicht gesättigt ist, so daß die Zusammensetzung des Dialysats variiert.

Ein Behältnis gemäßβ dem Oberbegriff des Anspruchs 1 ist aus EP-A-0 475 825 bekannt.

Aufgabe der vorliegenden Erfindung ist es, ein Behältnis an die Hand zu geben, bei dem sichergestellt ist, daß auch bei schwerer löslichen Komponenten die zur Dialysesitzung bereitgestellte Lösung gesättigt ist.

Erfindungsgemäß wird diese Aufgabe ausgehend von einem bekannten Behältnis dadurch gelöst, daß sowohl der Zulauf wie auch der Ablauf auf einer Seite des Behältnisses im Bodenbereich, angeordnet sind. Wesentlich ist es hier, daß sowohl Zulauf und Ablauf in Nachbarschaft des als Feststoff vorgelegten Dialysatkonzentrats liegen. Hierdurch wird sichergestellt, daß das frische Wasser die in fester Form vorliegenden Dialysatkonzentratteilchen umspült und aufgrund der dadurch bedingten turbulenten Strömung und Wirbelbildung zu einer schnellstmöglichen Lösung beiträgt. Die Anordnung des Auslaufs auf der gleichen Seite stellt sicher, daß die Flüssigkeit auch bei einem kontinuierlichen Betrieb eine möglichst lange Verweilzeit im noch nicht aufgelösten Dialysatkonzentrat hat, so daß hier eine Sättigung der Lösung auch bei schlecht lösenden Salzen erreicht werden kann. Aufgrund dieser Anordnung können auch die Dichteunterschiede ausgeglichen werden.

Aus dem US-Patent Nr. 5,385,564 ist bereits eine Lösung bekannt, in welcher ebenfalls in einem Behältnis die granulatförmige Dialysatkonzentration vorgelegt ist und bei der das Wasser über einen Anschluß in das Behältnis eingeleitet wird. Allerdings muß zunächst das Wasser vollständig in das Behältnis geleitet werden und nach entsprechendem Lösen des Konzentrats in dem Wasser wird das fertige Dialysat wieder durch die gleiche Öffnung im Behältnis ausgeleitet. Mit dieser Lösung ist ein kontinuierlicher Betrieb nicht möglich.

Bevorzugte Ausgestaltungen der Erfindung ergeben sich aus den sich an den Hauptanspruch anschließenden Unteransprüchen.

Demnach kann das Behältnis vorteilhaft in Form eines Beutels ausgebildet sein, der aus zwei zusammengeschweißten Folien gebildet ist, in dessen Bodenbereich der Zulauf bzw. Ablauf gebildet wird, wobei Zulauf und Ablauf vorzugsweise in Form von Konnektoren ausgebildet sind, die mit der Dialysiervorrichtung verbindbar sind.

Besonders vorteilhaft sind sowohl im Zulauf wie auch im Ablauf jeweils Filter mit einer Porosität von 50 bis 500 µm angeordnet.

Besonders vorteilhaft läßt sich das Behältnis einsetzen, wenn das Dialysatkonzentrat unter anderem Bicarbonat oder Natriumchlorid enthält.

Weitere Einzelheiten und Vorteile der Erfindung werden anhand eines in der Zeichnung dargestellten Ausführungsbeispieles näher erläutert. Es zeigen:
- Fig. 1:: eine schematische Darstellung einer ersten Ausführungsvariante der vorliegenden Erfindung,
- Fig. 2, 2a:: eine graphische Verdeutlichung des Einleitens von Wasser in einem Beutel gemäß Figur 1, teilweise vergrößert und
- Fig. 3: eine Ausführungsvariante der vorliegenden Erfindung in vereinfachter Schnittdarstellung. und
- Fig. 4:: ein Diagramm, in welchem die Flüssigkeit in Abhängigkeit der Tempera- tur über die Zeit dargestellt ist.

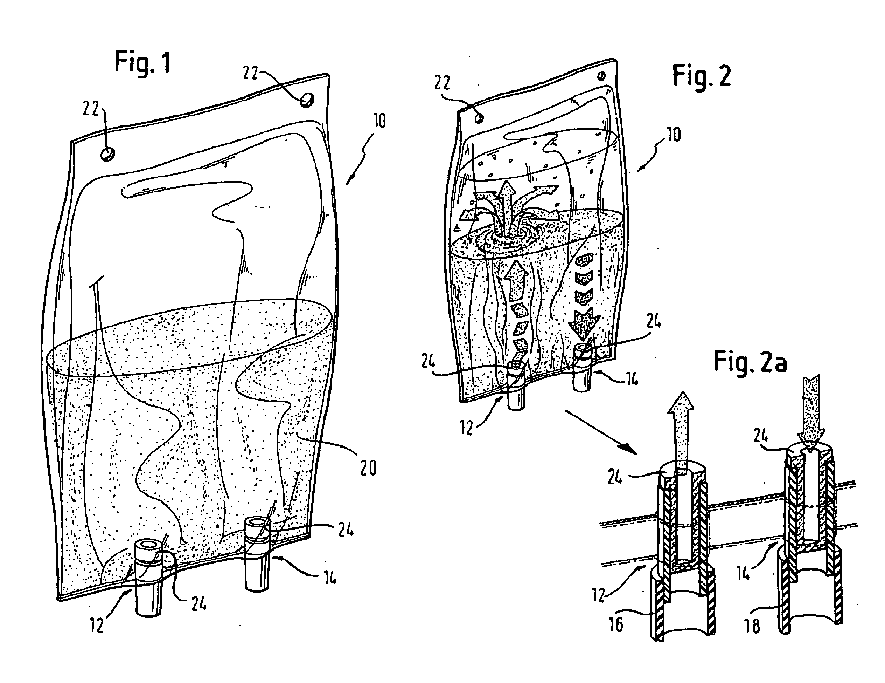

In der Ausführungsvariante gemäß Figur 1 besteht das Behältnis 10 aus einem Beutel, der aus zwei Kunststoffolien gefertigt ist, die seitlich miteinander verschweißt sind. Im Bodenbereich des Beutels 10 sind ein Einlaß 12 und ein Auslaß 14 vorgesehen, wobei diese im hier dargestellten Ausführungsbeispiel als rohrförmige Ansätze ausgebildet sind, auf die entsprechende Schlauchenden 16, 18 (vgl. Fig. 2a) aufschiebbar sind. Im unteren Teil des Beutels ist das aus Pulver, Granulat oder Pastillen bzw. Gemischen hiervon bestehende Dialysatkonzentrat 20 angeordnet. Im oberen Bereich weist der Beutel 10 zwei Aufnahmeöffnungen 22 auf, mittels derer dieser Beutel aufgehängt werden kann. Im Zulauf 12 bzw. Ablauf 14 sind jeweils in das Röhrchen einsteckbare Filter 24 mit einer Porosität von 50 bis 500 µm angeordnet. Im Schnitt sind diese Filter 24 ebenfalls in Figur 2a dargestellt.

In Figur 2 ist der Strömungsverlauf des durch den Zulauf 12 einströmenden Wassers gezeigt. Insbesondere ist hier durch die Pfeile angedeutet, daß entlang eines vergleichsweise breiteren Kanals das Dialysatkonzentrat durchströmt und dadurch wie auch durch die Dichteunterschiede zwischen konzentrierter und wenig konzentrierter Lösung auch verwirbelt wird. Von dem oberen Bereich des Beutels muß das Wasser nochmals das gesamte Haufwerk aus dem Dialysatkonzentrat durchströmen, um dann durch den Ablauf 14 in gesättigter Form abgezogen zu werden. Die Filter 24 verhindern jeweils ein Durchtreten von noch nicht aufgelöstem Dialysatkonzentrat in die Schlauchleitungen 16 bzw. 18.

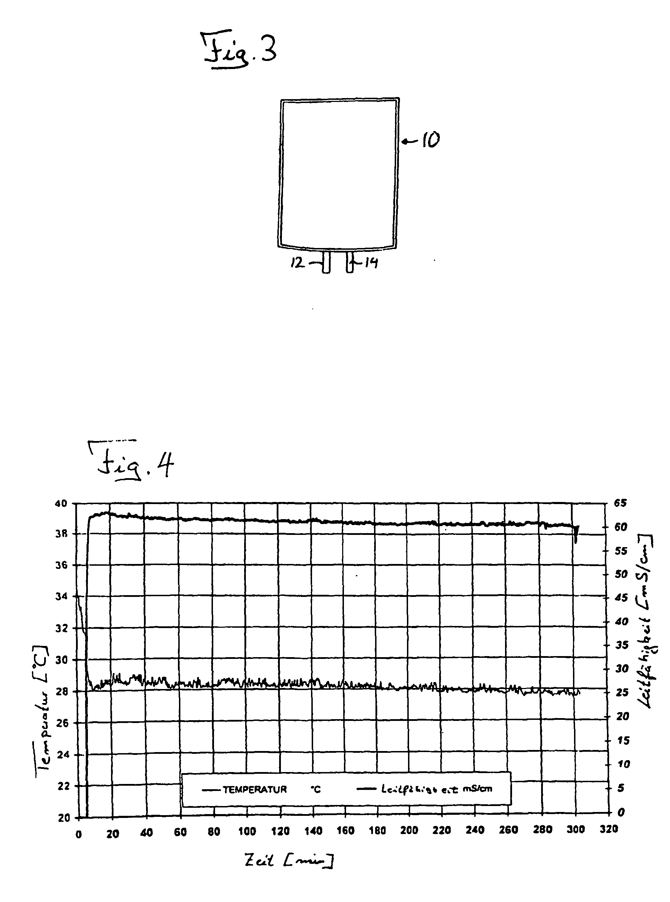

Anhand der Figur 3 kann eine Ausführungsvariant des erfindungsgemäßen Behältnisses 10, das hier Beutelform aufweist, erläutert werden. Dabei ist die Beutelform eine einfache Ausführungsform, die aber nicht zwingend gemäß der vorliegenden Erfindung vorgeschrieben ist.

Die Figur 3 zeigt einen Schnitt, der der Ausführungsform gemäß der Figuren 1 und 2 entspricht..

In Figur 4 ist ein Diagramm gezeigt, aus dem hervorgeht, daß bei konstanter Temperatur bei der Herstellung des Dialysats eine konstante Sättigungskonzentration erreichbar ist. Die Sättigung der Lösung wird hier durch Leitfähigkeitsmessung ermittelt. Es ergibt sich hier bei einer konstanten Temperatur von etwas mehr als 28°C eine ebenfalls konstante Leitfähigkeit von ca. 60 mS/cm, wobei hier das Dialysat am Auslauf 14 in eine Anordnung gemäß der Figur 1 über den entsprechenden Zeitraum gemessen wurde.

## Patentansprüche

1. Behältnis (10) zur Verwendung in der Dialyse enthaltend eine bestimmte Menge eines Salzkonzentrats in Form von Pulver, Granulat oder Pastillen oder Mischungen davon mit einem Zulauf (12) für das Wasser und mit einem an einem Dialysator anschließbaren Ablauf (14),
wobei sowohl Zulauf (12) wie auch Ablauf (14) auf einer Seite des Behältnisses (10) angeordnet sind,
**dadurch gekennzeichnet,**
**daß** sich im Inneren des Behältnisses (10) keine Trennwände befinden und daß sowohl Zulauf (12) wie auch Ablauf (14) so in einem Bodenbereich des Behältnisses (10) angeordnet sind, daß im Betrieb von unten aus dem Zulauf (12) durch das Dialysatkonzentrat (20) strömendes Wasser vom oberen Bereich des Behältnisses (10) aus nochmals das Dialysatkonzentrat (20) durchströmen muß, um durch den Ablauf (14) abgezogen zu werden.

2. Behältnis nach Anspruch 1, **dadurch gekennzeichnet, daß** es in Form eines Beutels (10) ausgebildet ist, der aus zwei zusammengeschweißten Folien gebildet ist, in dessen Bodenbereich den Zulauf (12) bzw. den Ablauf (14) bildende Konnektoren angeordnet sind.

3. Behältnis nach Anspruch 1 oder 2, **dadurch gekennzeichnet, daß** im Zulauf (12) bzw. Ablauf (14) jeweils ein Filter (24) mit einer Porosität von 50 bis 500 µm angeordnet ist.

4. Behältnis nach einem der Ansprüche 1 bis 3, **dadurch gekennzeichnet, daß** es unter anderem Bicarbonat oder Natriumchlorid enthält.

5. Verfahren zur Herstellung von Dialysat unter Verwendung eines Behältnisses (10) nach einem der vorangegangenen Ansprüche, wobei Wasser von unten aus dem Zulauf durch das Dialysatkonzentrat (20) strömt und vom oberen Bereich des Behälters aus nochmals das Dialysatkonzentrat (20) durchströmt, um durch den Ablauf abgezogen zu werden.

## Claims

1. A container (10) for use in dialysis containing a given amount of a salt concentrate in the form of powder, granules or pastilles or mixtures thereof, having a feed (12) for the water and a discharge (14) connectable to a dialyser,
wherein both the feed (12) and the discharge (14) are arranged on one side of the container (10)
**characterised in that**
there are no partitions in the interior of the container (10) and both the feed (12) and the discharge (14) are arranged in a bottom region of the container (10) so that in operation water flowing from below out of the feed (12) through the dialysate concentrate (20) has to flow out of the upper region of the container (10) through the dialysate concentrate (20) once again to be drawn off through the discharge (14).

2. A container according to claim 1 **characterised in that** it is in the form of a bag (10) formed from two films which are welded together, connectors forming the feed (12) and the discharge (14) respectively being arranged in the bottom region of the bag.

3. A container according to claim 1 or claim 2 **characterised in that** a respective filter (24) of a porosity of 50 to 500 µm is arranged in the feed (12) and the discharge (14) respectively.

4. A container according to one of claims 1 to 3 **characterised in that** it contains inter alia bicarbonate or sodium chloride.

5. A process for producing dialysate using a container (10) according to one of the preceding claims, wherein water flows from below out of the feed through the dialysate concentrate (20) and flows out of the upper region of the container through the dialysate concentrate (20) once again to be drawn off through the discharge.

## Revendications

1. Contenant (10) pour utilisation en dialyse, contenant une quantité définie d'un concentré de sel sous forme de poudre, granulés ou pastilles ou des mélanges de ceux-ci avec un afflux (12) pour l'eau et avec une évacuation (14) pouvant être raccordée à un dialyseur, où à la fois l'afflux (12) et aussi l'évacuation (14) sont disposés sur un côté du contenant (10),
**caractérisé en ce que**
à l'intérieur du contenant (10), il ne se trouve pas de paroi de séparation et qu'à la fois l'afflux (12) et aussi l'évacuation (14) sont disposés dans un fond du contenant (10) de telle sorte qu'en fonctionnement, l'eau du niveau inférieur sortant par l'évacuation (12) passant dans le concentré de dialyse (20) doive traverser à nouveau le concentré de dialyse (20) depuis la zone supérieure du contenant (10) pour être retirée par l'évacuation (14).

2. Contenant selon la revendication 1, **caractérisé en ce qu'**il est réalisé sous forme d'un sachet (10) qui est formé par deux feuilles assemblées par soudage, dans la zone de fond duquel sont disposés des connecteurs formant l'afflux (12) ou l'évacuation (14).

3. Contenant selon la revendication 1 ou 2, **caractérisé en ce qu'**est disposé dans l'afflux (12) ou l'évacuation (14) respectivement un filtre (24) avec une porosité de 50 à 500 µm.

4. Contenant selon l'une des revendications 1 à 3, **caractérisé en ce qu'**il contient entre autres du bicarbonate ou du chlorure de sodium.

5. Procédé de fabrication de dialysat en utilisant un contenant (10) selon l'une des revendications précédentes, dans lequel l'eau de la zone inférieure sortant par l'évacuation passe dans le concentré de dialyse (20) et traverse à nouveau le concentré de dialyse (20) depuis la zone supérieure du contenant pour être retirée par l'évacuation (14).
